(19)

Europäisches
Patentamt
European
Patent Office
Office européen
des brevets

(11)　EP 1 615 671 B1

(12)　EUROPEAN PATENT SPECIFICATION

(45) Date of publication and mention
of the grant of the patent:
21.05.2014　Bulletin 2014/21

(21) Application number: 04809310.8

(22) Date of filing: 08.03.2004

(51) Int Cl.:
A61K 51/00 (2006.01)　A61K 51/02 (2006.01)
A61K 51/12 (2006.01)　A61M 36/14 (2006.01)
A61P 35/00 (2006.01)　A61K 101/00 (2006.01)
A61K 103/00 (2006.01)　A61K 103/32 (2006.01)

(86) International application number:
PCT/US2004/007061

(87) International publication number:
WO 2005/035005 (21.04.2005 Gazette 2005/16)

(54) MICROSPHERES COMPRISING THERAPEUTIC AND DIAGNOSTIC RADIOACTIVE ISOTOPES

MIKROKÜGELCHEN MIT THERAPEUTISCHEN UND DIAGNOSTISCHEN RADIOAKTIVEN ISOTOPEN

MICROSPHERES CONTENANT DES ISOTOPES RADIOACTIFS THERAPEUTIQUES ET DIAGNOSTIQUES

(84) Designated Contracting States:
AT BE BG CH CY CZ DE DK EE ES FI FR GB GR
HU IE IT LI LU MC NL PL PT RO SE SI SK TR

(30) Priority: 04.04.2003　US 407144

(43) Date of publication of application:
18.01.2006　Bulletin 2006/03

(60) Divisional application:
12193933.4 / 2 574 347

(73) Proprietor: BIOSPHERE MEDICAL, INC.
Rockland, MA 02370-1052 (US)

(72) Inventors:
• SCHWARZ, Alexander
Brookline, MA 02446 (US)
• KROM, James, A.
Belmont, MA 02478 (US)

(74) Representative: Bradley, Adrian et al
Cleveland LLP
10 Fetter Lane
London EC4A 1BR (GB)

(56) References cited:
WO-A-02/34300　　US-A- 5 302 369

• NIJSEN J F W ET AL: "Advances in nuclear oncology: microspheres from internal radionuclide therapy of liver tumors" 1 January 2002 (2002-01-01), CURRENT MEDICINAL CHEMISTRY, BENTHAM SCIENCE PUBLISHERS BV, BE, PAGE(S) 73 - 82 , XP002983145 ISSN: 0929-8673 * the whole document *
• R.J. MUMPER ET AL: "Neutron-Activated Holmium-166-Poly (L-Lactic Acid) Microspheres: A Potential Agent for the Internal Radiation Therapy of Hepatic Tumors" JOURNAL OF NUCLEAR MEDICINE, vol. 32, no. 11, November 1991 (1991-11), pages 2139-2143, XP002531706
• U.O. HÄFELI ET AL: "Hepatic Tumor Radioembolization in a Rat Model Using Radioactive Rhenium (186RE/188RE) Glass Microspheres" INT. J. RADIATION ONCOLOGY BIOL. PHYS., vol. 44, no. 1, April 1999 (1999-04), pages 189-199, XP002531707
• S. HO ET AL: "Clinical Evaluation of the Partition Model for Estimating Radiation Doses from Yttrium-90 Microspheres in the Treatment of Hepatic Cancer" EUROPEAN JOURNAL OF NUCLEAR MEDICINE, vol. 24, no. 3, March 1997 (1997-03), pages 293-298, XP002531708
• ARIEL I M ET AL: "Therapeutic intralymphatic infusion of radioactive isotopes" 1 January 1963 (1963-01-01), JOURNAL OF NUCLEAR MEDICINE, SOCIETY OF NUCLEAR MEDICINE, RESTON, VA, US, PAGE(S) 186 , XP008106947 ISSN: 0161-5505 * Abstract D1 *

EP 1 615 671 B1

(Cont. next page)

- **NIJSEN J.F.W. ET AL.: 'Advances in nuclear oncology: microspheres from internal radionuclide therapy of liver tumors' CURRENT MEDICINAL CHEMISTRY vol. 9, 2002, pages 73 - 82, XP002983145**

## Description

### *Background of the Invention*

The development of new and more effective treatments for cancer is of utmost concern. This need is particularly urgent for the treatment of malignant tumors found in the liver owing to the current unsatisfactory treatment options. At the present time, the preferred method of treatment for patients with liver metastases is surgical resection. Unfortunately, the 5-year survival rate for patients that have undergone this form of treatment is only around 35%. Scheele J and Altendorf-Hofmann A. Resection of colorectal liver metastases. Langenbeck's Arch. Surg. 1999; 313-327. This disappointingly low survival rate is compounded by the fact that most tumours are inoperable by the time of diagnosis. Other treatment options for these tumours include conventional chemotherapy and external radiotherapy. Häfeli UO, Casillas S, Dietz DW, Pauer GJ, Rybicki LA, Conzone SD and Day DE. Hepatic tumor radioembolization in a rat model using radioactive rhenium (186Re/188Re) glass microspheres. Int. J. Radiation Oncology Biol. Phys. 1999; 44:189-199 and Link KH, Kornman M., Formentini A, Leder G, Sunelaitis E, Schatz M, Prelmar J and Beger HG. Regional chemotherapy of non-resectable liver metastases from colorectal cancer - literature and institutional review. Langenbeck's Arch. Surg. 1999; 384:344353. Unfortunately, neither of the latter regimens have shown significant improvements in patient survival.

Recent developments in selective radionuclide therapy indicate that radiolabeled microspheres may offer a promising treatment option for patients suffering from a variety of types of cancer. This treatment allows the selective delivery of therapeutic radioactive particles to the tumor with as little surrounding tissue damage as possible. This new treatment option is particularly important for cancers with an extremely poor prognosis and without other adequate therapies, such as primary and metastatic malignancies of the liver. For example, the regional administration of therapeutic agents via the hepatic artery is one strategy that has been developed to improve tumour response. Bastian P, Bartkowski R, Kohler H and Kissel T. Chemo-embolization of experimental liver metastases. Part 1: distribution of biodegradable microspheres of different sizes in an animal model for the locoregional therapy. Eur. J. Pharm. Biopharm. 1998; 46:243-254. This form of treatment promises to be particularly effective for both primary and metastatic liver cancer since these tumors are well vascularized and receive the bulk of their blood supply from the hepatic artery. Ackerman NB, Lien WM, Kondi ES and Silverman NA. The blood supply of experimental liver metastases. The distribution of hepatic artery and portal vein blood to "small" and "large" tumors. Surgery 1969; 66:1067-1072. In addition, many kinds of radiolabeled particles and radionuclides have been tested for local treatment of a variety of tumors in organs, including liver, lung, tongue, spleen and soft tissue of extremities.

In early applications of this technique, yttrium oxide powder was suspended in a viscous medium prior to administration. Yttrium oxide was selected for the technique because it emits nearly 100 percent beta radiation. See Nolan et al., Intravascular Particulate Radioisotope Therapy, The American Surgeon 1969; 35: 181-188 and Grady et al., Intra-Arterial Radioisotopes to Treat Cancer, American Surgeon 1960; 26:678-684. However, the yttrium oxide powder had a high density (5.01 gm/cm$^3$) and irregular particle shape. The high density of pure yttrium oxide powder made it difficult to keep the particles in suspension in the liquids used to inject them into the body, and the sharp corners and edges of yttrium oxide particles also irritate surrounding tissue in localized areas. In later applications, the particles used have been microspheres composed of an ion exchange resin, or crystalline ceramic core, coated with a radioactive isotope such as P-32 or Y-90. Both ion exchange resin and crystalline ceramic microspheres offer the advantage of having a density much lower than that of yttrium oxide particles, and the ion exchange resin offers the additional advantage of being particularly easy to label. See Zielinski and Kasprzyk, Synthesis and Quality Control Testing of 32P labelled Ion Exchange Resin Microspheres for Radiation Therapy of Hepatic Neoplasms, Int. J. Appl. Radiat. Isot. 1983; 34:1343-1350.

In still another application, microspheres have been prepared comprising a ceramic material and having a radioactive isotope incorporated into the ceramic material. While the release of radioactive isotopes from a radioactive coating into other parts of the human body may be eliminated by incorporating the radioisotopes into ceramic spheres, the latter product form is nevertheless not without its disadvantages. Processing of these ceramic microspheres is complicated because potentially volatile radioactivity must be added to ceramic melts and the microspheres must be produced and sized while radioactive, with the concomitant hazards of exposure to personnel and danger of radioactive contamination of facilities.

The current technology often uses glass, resin, albumin, or polymer microspheres that are impregnated with a material that emits β-particles upon neutron activation. Research has indicated that the composition of the bead can be important in the design of an effective treatment. For example, glass is relatively resistant to radiation-damage, highly insoluble, and non-toxic. Glass can be easily spheridized in uniform sizes and has minimal radionuclidic impurities. Advances in manufacturing have led to the production of glass microspheres with practically no leaching of the radioactive material. Ho S, Lau WY, Leung TWT, Chan M, Ngar YK, Johnson PJ and Li AKC. Clinical evaluation of the partition model for estimating radiation doses from yttrium-90 microspheres in the treatment of hepatic cancer. Eur. J Nucl. Med. 1997; 24:293-298.

Although glass spheres have several advantages, their high density (3.29 g/ml) and non-biodegradability are major drawbacks. Mumper RJ, Ryo UY and Jay M. Neutron activated holmium-166-Poly(L-lactic acid) microspheres: A potential agent for the internal radiation therapy of hepatic tumours. J. Nucl. Med. 1991; 32:2139-2143 and Turner JH, Claringbold PG, Klemp PFB, Cameron PJ, Martindale AA, Glancy RJ, Norman PE, Hetherington EL, Najdovski L and Lambrecht RM. 166Ho-microsphere liver radiotherapy: a preclinical SPECT dosimetry study in the pig. Nucl. Med. Comm. 1994; 15:545-553. The relatively high density increases the chance of intravascular settling. Ho S, Lau WY, Leung TWT and Johnson PJ. Internal radiation therapy for patients with primary or metastatic hepatic cancer. Cancer 1998; 83:1894-1907. Nevertheless, glass microspheres produced under the name TheraSpheres® were the first registered microsphere product for internal radionuclide therapy, and have been used in patients with primary or metastatic tumours. In comparison, only a few radioisotopes have the characteristics necessary for the treatment of tumors. Important characteristics of a suitable radioisotope would include a radiational spectrum (strength of $\beta$-particle emision) appropriate to the size of the tumor, high dose rate, short half-life, and $\gamma$-emission for external imaging.

The most suitable radioactive materials are yttrium-90, rhenium-188 and holmium-166. All three of these materials emit $\beta$-Radiation useful for radiotherapy. Although $^{90}$Y is often used in radionuclide therapy, yttrium-90 has two major disadvantages for use in radiotherapy. First, long neutron activation times (>2 weeks) are needed to achieve therapeutic activities of yttrium because $^{90}$Y's precursor has a small thermal neutron cross section of 1.28 barn. Secondly, the biodistribution of microspheres loaded with $^{90}$Y cannot be directly determined in clinical trials, since $^{90}$Y is a pure $\beta$-emitter and does not produce imageable $\gamma$-rays. Natural rhenium is composed of two isotopes, $^{185}$Re and $^{187}$Re, that form $\beta$-emitting $^{186}$Re and $^{188}$Re radioisotopes, respectively, upon neutron activation. The nuclear and dosimetric properties of the rhenium radioisotopes are comparable to those of $^{90}$Y, but they have imageable $\gamma$-photons. Like the rhenium radioisotopes, $^{166}$Ho emits $\beta$-particles and photons and has a relatively short physical half-life of 26.8 h, compared to $^{90}$Y (64.1 h) and $^{186}$Re (90.6 h), resulting in a high dose rate.

The development of microspheres for radionuclide therapy is complicated by the difficulty in determining the biodistribution of the microspheres in vivo, as noted above for $^{90}$Y. The biodistribution of microspheres is critically important for this type of radiotherapy because the microsphere must be in close proximity to the tumor being treated. One potential solution to this problem would be to attach a material to the microsphere that emits a detectable, non-hazardous signal.

*Summary of the Invention*

One aspect of the present invention relates to a microsphere, comprising a material selected from the group consisting of glass, polymer and resin; a first radioisotope that emits a therapeutic $\beta$-particle; and a second radioisotope that emits a diagnostic $\gamma$-ray; wherein the atomic number of the first radioisotope is not the same as the atomic number of the second radioisotope.

The present invention also relates to a method of preparing a radioactive microsphere, comprising the steps of: combining a non-radioactive precursor of a first radioisotope, a non-radioactive precursor of a second radioisotope, and a material selected from the group consisting of glass, polymer, and resin, to form a mixture; wherein the atomic number of the first radioisotope is not the same as the atomic number of the second radioisotope; fabricating a microsphere from said mixture; and bombarding said microsphere with neutrons.

Another aspect of the present invention relates to a method of treating a mammal suffering from a medical condition, comprising the step of administering to said mammal a therapeutically effective amount of radioactive microspheres each comprising a material selected from the group consisting of glass, polymer, and resin; a first radioisotope that emits a therapeutic $\beta$-particle; and a second radioisotope that emits a diagnostic $\gamma$-ray; wherein the atomic number of the first radioisotope is not the same as the atomic number of the second radioisotope.

*Detailed Description of the Invention*

The invention will be described more fully with reference to the accompanying examples, in which certain preferred embodiments of the invention are shown.

Radionuclide therapeutic techniques using microspheres for the treatment of various cancers rely upon the precise and accurate delivery of microspheres to a tumor. This treatment option offers the promise of delivering therapy directly to the tumor cells which minimizes damage to nearby healthy tissue, a serious shortcoming associated with conventional treatment options such as chemotherapy, radiotherapy, or surgical resection. However, the effectiveness of cancer treatments using radionuclide microspheres is often hampered by the inability to determine the biodistribution of the microspheres. Therefore, a non-invasive method to determine the biodistribution of said microspheres would be highly useful. Microspheres containing $^{198}$Au, for emission of $\gamma$-radiation to enable detection, have been designed that incorporate $^{90}$Y for emission of $\beta$-particles useful in the treatment of various medical conditions. A method to determine the amount of $^{197}$Au required per microsphere has been established based on the physical properties and relative

proportions of $^{90}$Y, $^{198}$Au, and the bulk material comprising the bead. A mathematical formula has been derived that allows for the calculation of the necessary quantity of the stable isotope of the therapeutic β-emitting radionuclide and $^{197}$Au. The composition and size of the microsphere may be customized to best fit a particular application. The radiolabeled microspheres may be introduced into a subject mammal in accord with standard procedures and the biodistribution of the microspheres may be determined by detection of the gamma-rays emitted by $^{198}$Au.

*Derivation of the Rate of Radioactive Decay*

Neutron activation of a stable isotope to give a radioactive isotope is described by the simple scheme where $k_N$ and $k_D$ are the neutron capture constant and radioactive decay constant, respectively.

$$A \xrightarrow{k_N} A^* \xrightarrow{k_D} P$$

*A, A\*,* and *P* represent the numbers of atoms (or moles) of the stable isotope, radioactive isotope, and decay product, respectively. The net rate of formation of the radioactive isotope *A\** is given by

$$\frac{dA^*}{dt} = k_N A - k_D A^* \qquad (Eq\ 1)$$

which has the solution, assuming no A\* is present initially,

$$A^* = \frac{k_N A_0}{k_D . k_N}(e^{k_N t} - e^{k_D t}) \qquad (Eq\ 2)$$

where $A_0$ is the initial quantity of the stable isotope.

The radioactive decay constant can be expressed in terms of the radioactive isotope's half-life ($t_{1/2}$) according to:

$$k_D = \frac{\ln(2)}{t_{1/2}} \qquad (Eq\ 3)$$

The neutron capture constant is determined by the neutron flux φ and the neutron capture cross-section χ according to:

$$k_N = \varphi \chi \qquad (Eq\ 4)$$

Constants φ and χ are typically expressed in units of cm$^{-2}$s$^{-1}$ and 10$^{-24}$cm$^2$ (barns), respectively.
After removing the material from the neutron source, the radioactive isotope will decay at a rate given by

$$\frac{dA^*}{dt} = - k_D A^* \qquad (Eq\ 5)$$

_Determination of Required Quantity of Stable Isotope_

The radioactivity, defined by _-dA\*/dt,_ is usually expressed in units of $s^{-1}$ (becquerel) or $3.7 \times 10^{10}$ $s^{-1}$ (curie). From Eq 2 and Eq 5, the equation that expresses the radioactivity at the time of removal of the sample from the neutron source is

$$-\frac{dA^*}{dt} = \frac{k_D k_N A_0 (e^{-k_N t} - e^{-k_D t})}{k_D - k_N} \qquad \text{(Eq 6)}$$

Solving Eq 6 for $A_0$ allows calculation of the quantity of stable isotope that is required to achieve a desired radioactivity after an irradiation time _t_ according to:

$$A_0 = \frac{dA^*}{dt} \frac{k_D - k_N}{k_D k_N (e^{-k_N t} - e^{-k_D t})} \qquad \text{(Eq 7)}$$

_Identity of β-Emitting Therapeutic Radionuclide_

A radionuclide suitable for internal radionuclide therapy of primary and metastatic malignancies must have the following properties: First, the radioisotope must have an appropriate radiation spectrum for treating small to large multiple tumours. Large tumours with a vascular periphery but a necrotic centre take up less microspheres per volume; therefore, a high energy β-emitter with a subsequently high tissue range is needed to reach the interior of the tumour. Second, a high dose rate is advantageous for the radiobiological effect. Spencer RP. Applied principles of radiopharmaceutical use in therapy. Nucl. Med. Biol. 1986; 13:461-463 and Spencer RP. Short-lived radionuclides in therapy. Nucl. Med. Biol. 1987; 14:537-538. Consequently, a short half life is preferable. Third, a γ-emitter is desirable for external imaging to determine the biodistribution of the radioisotope with a gamma camera. However, the radioactivity should be low to prevent unnecessary radiation burden to the patient and environment. Mumper RJ, Ryo UY and Jay M. Neutron activated holmium-166-Poly(L-lactic acid) microspheres: A potential agent for the internal radiation therapy of hepatic tumours. J. Nucl. Med. 1991; 32:2139-2143. Further, the labeling of particles has to be simple without any leakage of the isotope. Finally, a large thermal neutron cross section is needed to enable high specific activities to be achieved within short neutron activation times. Conzone SD, Häfeli UO, Day DE and Ehrhardt GJ. Preparation and properties of radioactive rhenium glass microspheres intended for in vivo radioembolization therapy. J. Biomed. Mater. Res. 1998; 42:617-625. Unfortunately, only a few radioisotopes have characteristics which make them potentially suitable for the treatment of tumours, such as $^{90}$Y, $^{99m}$Tc, $^{188}$Re, $^{32}$P, $^{166}$Ho, $^{109}$Pd, $^{140}$La, $^{153}$Sm, $^{165}$Dy, and $^{169}$Er.

_Detection of γ Photons Emitted by Diagnostic Radionuclide_

Today, cancer is often found using a gamma camera, which provides images of potential tumors in the body by detecting the radiation emitted by a radiopharmaceutical given to a patient undergoing a full-body scan. In such systemic approaches, suspected tumor regions collect higher concentrations of the radiopharmaceutical, which produces a higher count rate and therefore a detectable contrast between the tumor region and its surroundings.

Most clinically-used radiopharmaceuticals are diagnostic agents incorporating a gamma-emitting nuclide which, because of physical or metabolic properties of its coordinated ligands, localizes in a specific organ after intravenous injection. The resultant images can reflect organ structure or function. These images are obtained by means of a gamma camera that detects the distribution of ionizing radiation emitted by the radioactive molecules. The principal isotope currently used in clinical diagnostic nuclear medicine is metastable technetium-99m, which has a half-life of 6 hours.

As outlined above, a gamma camera is used in nuclear medicine for the display, in an organ, of the distribution of molecules marked by a radioactive isotope injected into a patient. Thus, a gamma camera has a collimator to focus the gamma photons emitted by the patient's body, a scintillator crystal to convert the gamma photons into light photons or scintillations, and an array of photomultiplier tubes, each of which converts the scintillations into electrical pulses. A detection system such as this is followed by a processing and display unit that can be used to obtain an image projection of the distribution of the radioactive isotopes in the patient during the acquisition of the image.

*Activation of Radionuclide*

A variety of neutron sources, such as reactors, accelerators, and radioisotopic neutron emitters, can be used for radioactivation of stable isotopes by neutron activation. Systems and methods for neutron activation are described in U.S. Patents Nos. 6,149,889 and 6,328,700. Nuclear reactors with their high fluxes of neutrons from uranium fission offer the highest available activation rates for most elements. Different types of reactors and different positions within a reactor vary considerably with regard to their neutron energy distributions and fluxes due to the materials used to moderate (or reduce the energies of) the primary fission neutrons. However, most neutron energy distributions are quite broad and include three principal components (thermal, epithermal, and fast).

The thermal neutron component includes low-energy neutrons (energies below 0.5 eV) in thermal equilibrium with atoms in the reactor's moderator. At room temperature, the energy spectrum of thermal neutrons is best described by a Maxwell-Boltzmann distribution with a mean energy of 0.025 eV and a most probable velocity of 2200 m/s. In most reactor irradiation positions, 90-95% of the neutrons that bombard a sample are thermal neutrons.

The epithermal neutron component includes neutrons (energies from 0.5 eV to about 0.5 MeV) which have been only partially moderated. A cadmium foil 1 mm thick absorbs all thermal neutrons, but will allow epithermal and fast neutrons above 0.5 eV in energy to pass through. In a typical unshielded reactor irradiation position, the epithermal neutron flux represents about 2% the total neutron flux. Both thermal and epithermal neutrons induce reactions on target nuclei.

The fast neutron component of the neutron spectrum (energies above 0.5 MeV) includes the primary fission neutrons which still have much of their original energy following fission. Fast neutrons contribute very little to the reaction, but instead induce nuclear reactions where the ejection of one or more nuclear particles - (n,p), (n,n'), and (n,2n) - are prevalent. In a typical reactor irradiation position, about 5% of the total flux consists of fast neutrons.

The amount of radiation delivered to a target by radioactive metal or metal compound labeled microspheres can be controlled in a variety of ways; for example, by varying the amount of metal associated with the spheres, the extent of radioactivation of the metal, the quantity of microspheres administered, and the size of microspheres administered.

*Bulk Composition of Microspheres*

*Glass*

Glass is relatively resistant to radiation-damage, highly insoluble, and non-toxic. Glass can be easily spheridized in uniform sizes and has minimal radionuclidic impurities. Advances in technology have led to the production of glass microspheres with practically no leaching of radioactive material. Ho S, Lau WY, Leung TWT, Chan M, Ngar YK, Johnson PJ and Li AKC. Clinical evaluation of the partition model for estimating radiation doses from yttrium-90 microspheres in the treatment of hepatic cancer. Eur. J Nucl. Med. 1997; 24:293-298. Although the glass spheres have several advantages, their high density (3.29 g/ml) and their non-biodegradability are drawbacks. Mumper RJ, Ryo UY and Jay M. Neutron activated holmium-166-Poly(L-lactic acid) microspheres: A potential agent for the internal radiation therapy of hepatic tumours. J. Nucl. Med. 1991; 32:2139-2143. The relatively high density of glass increases the chance of intravascular settling. Glass microspheres produced under the name TheraSpheres® were the first registered microsphere product for internal radionuclide therapy, and are used in patients with primary or metastatic tumours. Because of the lack of y-emission of $^{90}Y$, radioactive rhenium ($^{186}Re/^{188}Re$) microspheres were also produced. The general method of manufacture of these spheres was the same as for the $^{90}Y$ spheres. Häfeli UO, Casillas S, Dietz DW, Pauer GJ, Rybicki LA, Conzone SD and Day DE. Hepatic tumor radioembolization in a rat model using radioactive rhenium (186Re/188Re) glass microspheres. Int. J. Radiation Oncology Biol. Phys. 1999; 44:189-199 and Conzone SD, Häfeli UO, Day DE and Ehrhardt GJ. Preparation and properties of radioactive rhenium glass microspheres intended for in vivo radioembolization therapy. J. Biomed. Mater. Res. 1998; 42:617-625.

Brown et al. prepared $^{166}Ho$-loaded glass particles for direct injection into tumours of mice, which resulted in an effective modality for deposition of intense $\gamma$-radiation for use in localized internal radionuclide therapy; however, no further studies were done. Brown RF, Lindesmith LC and Day DE. 166-Holmium-containing glass for internal radiotherapy of tumors. Int. J. Rad. Appl. Instrum. B 1991; 18:783-790.

Kawashita et al. suggested the use of phosphorus-rich $Y_2O_3$-$Al_2O_3$-$SiO_2$-glass microspheres containing phosphorus ions, which were produced by thermoelectron bombardment of red phosphorus vapour and implanted into glass, thus resulting in a high phosphorus content and high chemical durability. After activation by neutron bombardment the glass contains phosphorus-32 ($^{32}P$). Kawashita M, Miyaji F, Kokubo T, Takaoka GH, Yamada I, Suzuki Y and Inoue M. Surface structure and chemical durability of P+-implanted Y2O3-Al2O3-SiO2 glass for radiotherapy of cancer. J. Non-Cryst. Solids 1999; 255:140-148.

*Resins*

Resin-based microspheres are favoured for radio-embolization. Chloride salts of holmium and yttrium have been added to cation exchange resins. Different resins were investigated by Schubiger et al., amongst which were Bio-Rex 70, Cellex-P, Chelex 100, Sephadex SP and AG 50W-X8. Schubiger PA, Beer H-F, Geiger L, Rösler H, Zimmerman A, Triller J, Mettler D and Schilt, W. 90Y-resin particles-animal experiments on pigs with regard to the introduction of superselective embolization therapy. Nucl. Med. Biol. 1991; 18:305-311. The resins with $^{90}$Y bound to the carboxylic acid groups of the acrylic polymer were sterilized and used for renal embolization of pigs. Only the pre-treated Bio-Rex 70 resulted in usable particles, with a retention of beta activity in the target organ of >95% of injected dose, and no histologically detectable particles in lung tissue samples. Zimmerman A, Schubiger PA, Mettler D, Geiger L, Triller J and Rösler H. Renal pathology after arterial yttrium-90 microsphere administration in pigs. A model for superselective radio-embolization therapy. Invest. Rad. 1995; 30:716-723.

Aminex resins (Bio-Rad Inc. Hercules CA, USA) loaded with $^{166}$Ho or $^{188}$Re also resulted in usable preparations. Turner et al. prepared microspheres by addition of [166]Hochloride to the cation exchange resin Aminex A-5, which has sulphonic acid functional groups attached to styrene divinylbenzene copolymer lattices. Turner JH, Claringbold PG, Klemp PFB, Cameron PJ, Martindale AA, Glancy RJ, Norman PE, Hetherington EL, Najdovski L and Lambrecht RM. 166Ho-microsphere liver radiotherapy: a preclinical SPECT dosimetry study in the pig. Nucl. Med. Comm. 1994; 15:545-553. Reproducible, non-uniform distributions of the $^{166}$Ho-microspheres throughout the liver were observed on scintigraphic images, following intrahepatic arterial administration in pigs. This predictable distribution allowed these investigators to determine the radiation absorbed dose from a tracer activity of $^{166}$Ho-microspheres, and to define the administered activity required to provide a therapeutic dose. Aminex A-27 was labelled with $^{188}$Re by adding [188]Reperrhenate and SnCl$_2$ to vacuum-dried resin particles. Wang S-J, Lin W-Y, Chen M.-N, Chi C-S, Chen J-T, Ho W-L, Hsieh B-T, Shen L-H, Tsai Z-T, Ting G, Mirzadeh S and Knapp FF. Intratumoral injection of rhenium-188 microspheres into an animal model of hepatoma. J Nucl. Med. 1998; 39:1752-1757. The mixture was boiled and centrifuged and microspheres were separated and resuspended in saline. Spheres were tested by direct intratumoural injection into rats with hepatoma. Survival over 60 days was significantly better in the treated versus the control group (80% vs. 27%).

Investigators from Australia and Hong Kong have used unspecified resin-based particles labeled with $^{90}$Y for treatment of patients with primary or secondary liver cancer. Lau WY, Leung WT, Ho S, Leung NWY, Chan M, Lin J, Metreweli C, Johnson P and Li AKC. Treatment of inoperable hepatocellular carcinoma with intrahepatic arterial yttrium-90 microspheres: a phase I and II study. Br. J. Cancer 1994; 70:994-999. The spheres had a diameter of 29-35 $\mu$m, a density of 1.6 g/mL and a specific activity of approximately 30-50 Bq per sphere. Treatment was well tolerated with no bone-marrow or pulmonary toxicity. The median survival was 9.4 months (range 1.8-46.4) in 71 patients, and the objective response rate in terms of drop in tumour marker levels was higher than that based on reduction in tumour volume shown by computed tomography. Lau WY, Ho S, Leung TWT, Chan M, Ho R, Johnson PJ and Li AKC. Selective internal radiation therapy for nonresectable hepatocellular carcinoma with intraarterial infusion of 90yttrium microspheres. Int. J. Radiation Oncology Biol. Phys. 1998; 40:583592.

*Albumin*

Since 1969, technetium-99m-microspheres ($^{99m}$Tc-microspheres) of human serum albumin (HSA) have been widely used for clinical nuclear medicine, particularly for lung scanning. Wunderlich G, Pinkert J, Andreeff M, Stintz M, Knapp FF, Kropp J and Franke WG. Preparation and biodistribution of rhenium-188 labeled albumin microspheres B 20: a promising new agent for radiotherapy. Appl. Radiat. Isotopes 2000; 52:63-68 and Rhodes BA, Zölle I, Buchanan JW and Wagner HN. Radioactive albumin microspheres for studies of the pulmonary circulation. Radiology 1969; 92:1453-1460. $^{188}$Re-labeled HSA microspheres used by Wunderlich et al. are uniform in size, with a mean diameter of 25 $\mu$m, and are biocompatible and biodegradable. However, the labeling process is time-consuming and depends on SnCl$_2$.2H$_2$O and gentisic acid concentration. On the surface of the microspheres a shell of less than about 1 $\mu$m thickness was seen, probably consisting of precipitated tin hydroxide. The particle labeling (coating) may be achieved by a combination of the reduction reaction of Re(VII) with Sn(II) and a particle surface-related coprecipitation effect of tin hydroxide colloid with high adsorption capacity and reduced, hydrolysed rhenium. The labeling yield under optimal reaction conditions is more than 70%. Biodistribution experiments in rats, using the lungs as a model for a well-perfused tumour, resulted in excellent in vivo stability.

As well as rhenium, yttrium has been bound to HSA for internal radiotherapy. Watanabe N, Oriuchi N, Endo K, Inoue T, Tanada S, Murata H and Sasaki Y. Yttrium-90 labeled human macroaggregated albumin for internal radiotherapy: combined use with DTPA. Nucl. Med. Biol. 1999; 26:847-851. $^{90}$Y-acetate and macroaggregates of HSA (MAA) (Macrokit®, Dainabot, Tokyo, Japan) were suspended in sodium acetate buffer and incubated at room temperature. Experiments in mice were carried out in order to investigate the possibility of using $^{90}$Y-MAA as an internal radiotherapeutic agent for whole-lung irradiation. Yttrium-activity in the lung was cleared within 72 h post injection and activity was redistributed in other organs, especially in the bone, but this could be prevented by the combined use of $CaNa_3DTPA$. Based on its rapid clearance $^{90}$Y-MAA was suggested as being useful for fractionated internal radiotherapy of the lung.

*Polymers*

Polymer-based microspheres have many advantages over other materials, in particular their near-plasma density, biodegradability and biocompatibility. However, the major disadvantage is their inability to withstand high thermal neutron fluxes. Conzone SD, Häfeli UO, Day DE and Ehrhardt GJ. Preparation and properties of radioactive rhenium glass microspheres intended for in vivo radioembolization therapy. J. Biomed. Mater. Res. 1998; 42:617-625. Additives and adjustment of irradiation-parameters can overcome this problem. A solvent evaporation technique has been used for preparation of poly(L-lactic acid) (PLLA) microspheres containing $^{166}$Ho, $^{90}$Y and $^{186}$Re/[188]Re. Mumper et al. has prepared PLLA microspheres with holmium-165-acetylacetonate (HoAcAc). Mumper RJ and Jay M. Poly(L-lactic acid) microspheres containing neutron-activatable holmium-165: A study of the physical characteristics of microspheres before and after irradiation in a nuclear reactor. Pharm. Res. 1992; 9:149-154. HoAcAc complex and PLLA were dissolved in chloroform and the solution was added to a polyvinyl alcohol (PVA) solution and stirred until the solvent had evaporated. Microspheres were graded and collected according to size, on stainless steel sieves having 20-50 $\mu$m openings. These microspheres can be dispensed in patient-ready doses that only need to be activated by neutron bombardment to a therapeutic amount of radioactivity in a nuclear reactor. These holmium loaded microspheres are currently being tested by intrahepatic arterial administration to rat liver tumours. A seven-fold increase of the $^{166}$Ho microspheres in and around the tumour compared with normal liver was found, based on distribution of radioactivity.

Magnetic PLLA microspheres loaded with yttrium were made by Hafeli et al. in order to direct them to the tumour. Häfeli UO, Sweeney SM, Beresford BA, Humm JL and Macklis RM. Effective targeting of magnetic radioactive 90Y-microspheres to tumor cells by an externally applied magnetic field. Preliminary in vitro and in vivo results. Nucl. Med. Biol. 1995; 22:147-155. This method resulted in stably loaded spheres, with the possibility of pre- or afterloading. To produce preloaded microspheres, PLLA was dissolved with L-$\alpha$-phosphatidylcholine in methylene chloride. Commercially available [90]$YCl_3$ and magnetite $Fe_3O_4$ were added to the solution, vortexed, and sonicated. The suspension was injected into PBS with PVA, and microspheres were prepared following a solvent evaporation technique. Afterloaded spheres were prepared by suspending dried microspheres in a solution of PBS, after which [90]$YCl_3$ in HCl was added. Spheres were subsequently vortexed, incubated, and washed, resulting in labeled microspheres. Leaching of $^{90}$Y was around 4% after 1 day in PBS at 37°C. Specific activity was 1.85 MBq/mg in both methods. $^{90}$Y was bound to the carboxylic endgroups of the PLLA. Experiments in mice showed a 12-fold increase in activity in the tumour with a directional magnet fixed above it. Rhenium loaded PLLA microspheres were also developed, but these microspheres were unable to withstand the high neutron fluxes in a nuclear reactor which are necessary to achieve the high specific activity required in the treatment of liver tumours. Häfeli UO, Casillas S, Dietz DW, Pauer GJ, Rybicki LA, Conzone SD and Day DE. Hepatic tumor radioembolization in a rat model using radioactive rhenium (186Re/188Re) glass microspheres. Int. J. Radiation Oncology Biol. Phys. 1999; 44:189-199.

*Manufacture of Microspheres*

In certain cases, such as described in U.S. Patent 5,302,369, microspheres have been prepared from a homogenous mixture of powders (i.e., the batch) that is melted to form the desired glass composition. The exact chemical compounds or raw materials used for the batch is not critical so long as they provide the necessary oxides in the correct proportion for the melt composition being prepared. For instance, if a YAS glass is being made, then yttria, alumina, and silica powders could be used as the batch raw materials. The purity of each raw material is preferably greater than 99.9%. After either dry or wet mixing of the powders to achieve a homogeneous mixture, the mixture may be placed in a platinum crucible for melting. High purity alumina crucibles can also be used if at least small amounts of alumina can be tolerated in the glass being made. The crucibles containing the powdered batch are then placed in an electric furnace which is heated 1500° to 1600° C, depending upon the composition. In this temperature range, the batch melts to form a liquid which is stirred several times to decrease its chemical heterogeneity. The melt should remain at 1500° to 1600° C until all solid material in the batch is totally dissolved, usually 2-5 hours being sufficient. When melting and stirring is complete, the crucible is removed from the furnace and the melt is quickly quenched to a glass by pouring the melt onto a cold steel plate or into clean water. This procedure breaks the glass into fragments, which aids and simplifies crushing the glass to a fine powder. The powder is then sized and spheroidized for use.

Where it is desired to use microspheres having a diameter in the range of about 20 to about 30 micrometers, as for in the treatment of liver cancer, it is preferred that the quenched and broken glass be first crushed to about minus 100 mesh particles using a mortar and pestle. The minus 100 mesh material is then ground using a mechanized mortar and pestle or ball mill, until it passes a 400 mesh sieve. The particles are formed into glass microspheres by introducing the -400 mesh particles into a gas/oxygen flame where they are melted and a spherical liquid droplet is formed by surface tension. The droplets are rapidly cooled before they touch any solid object so that, their spherical shape is retained in the solid product.

Just prior to spheroidizing, the -400 mesh powder is rescreened through a 400 mesh sieve to remove any large agglomerates that may have formed during storage. The -400 mesh powder is then placed in a vibratory feeder located above the gas/oxygen burner. The powder is slowly vibrated into a vertical glass tube which guides the falling powder particles directly into the hot flame of a gas/oxygen burner. Any burner capable of melting -400 mesh particles of the particular glass composition being used is satisfactory. A typical rate for feeding the powder to the flame is 5 to 25 gm/hr with the described apparatus. The flame of the burner is directed into a metal container which catches the small glass beads as they are expelled from the flame. This container can be made of any metal which can withstand the heat of the burner and does not contaminate the glass. The container needs to be large enough so that the molten spheres can cool and become rigid before hitting a solid surface.

After spheroidization, the glass spheres are collected and rescreened. When the microspheres are intended to be used in the treatment of liver cancer, the fraction less than 30 and greater than 20 micrometers in diameter is recovered since this is the desirable size for use in the human liver. After screening, the -30/+20 microspheres are examined with an optical microscope and are then washed with a weakly acidic solution, filtered, and washed several times with reagent grade acetone. The washed spheres are then heated in a furnace in air to 500°-600° C for 2-6 hours to destroy any organic material.

The final step is to examine a representative sample of the -30/+20 spheres in a scanning electron microscope to evaluate the size range and shape of the spheres. The quantity of undersize spheres (less than 10 micrometers in diameter) is determined along with the concentration of non-spherical particles. The composition of the spheres can be checked by energy dispersive x-ray analysis to confirm that the composition is correct and that there is an absence of chemical contamination. The glass microspheres are then ready for irradiation and subsequent administration to the patient.

Polymer-based microspheres used for internal radionuclide therapy are mainly prepared by a solvent evaporation technique. In the solvent evaporation process, the polymer is dissolved in a suitable water immiscible volatile solvent, and the medicament is dispersed or dissolved in this polymeric solution. The resulting solution or dispersion is then emulsified by stirring in an aqueous continuous phase, thereby forming discrete droplets. In order that the microspheres should form, the organic solvent must first diffuse into the aqueous phase and then evaporate at the water/air interface. As solvent evaporation occurs the microspheres harden, and free flowing microspheres can be obtained after suitable filtration and drying. O'Donnell PB and McGinity JW. Preparation of microspheres by solvent evaporation technique. Adv. Drug Del. Rev. 1997; 28:25-42

*Administration of Microspheres*

The microspheres may be administered to the patient through the use of catheters either alone or in combination with vasoconstricting agents or by any other means of administration that effectively causes the microspheres to become embedded in the cancerous or tumor bearing tissue. See U.S. Patent 5,302,369. For purposes of administration, the microspheres are preferably suspended in a medium that has a sufficient density or viscosity that prevents the microspheres from settling out of suspension during the administration procedure. Presently, preferred liquid vehicles for suspension of the microspheres include polyvinylpyrrolidone (PVP), sold under the trade designation Plasdone K-30 and Povidone by GAF Corp, a contrast media sold under the trade designation Metrizamide by Nyegard & Co. of Oslo, Norway, a contrast media sold under the trade designation Renografin 76 by E. R. Squibb & Co., 50% dextrose solutions and saline.

*Selected Clinical Applications of Radionuclide Microspheres*

Given the increased skills of interventional radiologists, there is increasing interest in selective radionuclide therapy. Many kinds of radiolabeled particles and radionuclides have been tested for local treatment of a variety of tumours in organs, including liver, lung, tongue, spleen and soft tissue of extremities. The purpose of this treatment is the superselective application of suitable radioactive (high energetic $\beta$-emitters) particles to deliver high doses to the tumour, with as little surrounding tissue damage as possible. These new treatment methods are promising particularly for cancers with a poor prognosis and without other adequate therapies, such as primary and metastatic malignancies of the liver.

*Liver cancer*

Patients with primary or metastatic tumours were treated by radio-embolization via a catheter or direct injection of beads into the tumour with a needle. Gray BN, Burton MA, Kelleher D, Klemp P and Matz L. Tolerance of the liver to the effects of yttriuin-90 radiation. Int. J. Radiation Oncology Biol. Phys. 1990; 18:619-623 and Tian J-H, Xu B-X, Zhang J-M, Dong B-W, Liang P and Wang X-D. Ultrasoundguided internal radiotherapy using yttrium-90-glass microspheres for liver malignancies. J. Nucl. Med. 1996; 37:958-963. Most studies describe administration of microspheres to patients via a catheter, whereby the tip was placed in the hepatic artery. The spheres eventually lodge in the microvasculature of the liver and tumour, remaining until the complete decay of the radioisotope. Lung shunting and tumour-to-normal liver ratio was determined after infusion of $^{99m}$Tc-labeled macroaggregated albumin, and microspheres were subsequently administered to patients. Ho S, Lau WY, Leung TWT, Chan M, Chan KW, Lee WY, Johnson PJ and Li AKC. Tumour-to-normal ratio of 90Y microspheres in hepatic cancer assessed with 99mTc macroaggregated albumin. Brit. J. Rad. 1997; 70:823-828. Tumour-to-normal liver ratio was approximately 3-5. Yorke ED, Jackson A, Fox RA, Wessels BW and Gray N. Can current models explain the lack of liver complications in Y-90 microsphere therapy? Clin. Cancer Res. 1999; 5:3024s-3030s. In some studies the blood flow within the liver was temporarily redirected in favour of the tumour by a bolus infusion of a vasoconstrictor, and the spheres were then embolized into the arterial circulation. While external beam radiation causes radiation hepatitis at doses above 30-35 Gy the liver can tolerate up to 80-150 Gy, using internal radionuclide therapy. Ingold J, Reed G, Kaplan H and Bagshaw M. Radiation hepatitis. Am. J. Roentgenol. Radium Ther. Nucl. Med. 1965; 93:200-208. Increased longevity, pain relief, tumour response and total clinical improvement are frequently reported.

*Head and neck cancers*

Chemo-embolization with ethylcellulose microspheres of 100-450 $\mu$m has been used in the treatment of maxillary tumours. The role of intra-arterial radioisotope therapy in the treatment of head and neck cancer is just beginning in rabbits, in the work of van Es et al. Van Es RJJ, Franssen O, Dullens HFJ, Bernsen MR, Bosman F, Hennink WE and Slootweg PJ. The VX2 carcinoma in the rabbit auricle as an experimental model for intra-arterial embolization of head neck squamous cell carcinoma with hydrogel dextran microspheres. Lab. Anim. 1999; 33:175-184. The optimal size of microspheres for treatment of unresectable head-and-neck cancer is still to be established. Some embolizations in the treatment of head-and-neck cancer have been carried out with particles of 100-450 pm. Tomura N, Kato K, Hirano H, Hirano Y and Watarai J. Chemoembolization of maxillary tumors via the superficial temporal artery using a coaxial catheter system. Radiation Med. 1998; 16:157.

*Other cancers*

Intra-arterial administration of $^{90}$Y-microspheres has been carried out in the spleen. Ariel IM and Padula G. Irradiation of the spleen by the intra-arterial administration of 90yttrium microspheres in patients with malignant lymphoma. Cancer 1972; 31:90-96. Of nine patients with lymphosarcoma, five manifested no clinical response after splenic irradiation. One patient who complained of weakness, rapid fatigue and anorexia, had relief of all symptoms after splenic irradiation

*Definitions*

For convenience, certain terms employed in the specification, examples, and appended claims are collected here.
The articles "a" and "an" are used herein to refer to one or to more than one (i.e. to at least one) of the grammatical object of the article. By way of example, "an element" means one element or more than one element.
The term "radionuclide" refers to a radioactive isotope or element.
The term "biodistribution" refers to the location of the given particle or particles in a biological entity.
The term "microsphere" refers to an object that is substantially spherical in shape and has a diameter less than 1 millimeter.
The term "glass" refers to a hard, brittle, non-crystalline, inorganic substance, which is usually transparent; glasses are often made by fusing silicates with soda, as described by Webster's New World Dictionary. Ed. Guralnik, DB 1984.
The phrase "time of use" refers to the period during which a microsphere is implanted in a patient or subject.
For purposes of this invention, the chemical elements are identified in accordance with the Periodic Table of the Elements, CAS version, Handbook of Chemistry and Physics, 67th Ed., 1986-87, inside cover.

*Microspheres of the Invention*

The present invention relates to a microsphere, comprising a material selected from the group consisting of glass, polymer and resin; a first radioisotope that emits a therapeutic $\beta$-particle; and a second radioisotope that emits a

diagnostic $\gamma$-ray; wherein said first radioisotope is $^{90}$Y or $^{32}$P; said second radioisotope is $^{198}$Au; and wherein the ratio of the radioactivity of the second radioisotope to the first radioisotope is in the range from 1:10 to $1:10^7$.

In one embodiment, the present invention relates to the aforementioned microsphere, wherein the ratio of the radioactivity of the second radioisotope to the first radioisotope is in the range from $1:10^2$ to $1:10^6$.

In another embodiment, the present invention relates to the aforementioned microsphere, wherein the ratio of the radioactivity of the second radioisotope to the first radioisotope is in the range from $1:10^4$ to $1:10^5$.

In another embodiment, the present invention relates to the aforementioned microsphere, wherein said material is selected from the group consisting of glass and polymer.

In another embodiment, the present invention relates to the aforementioned microsphere, wherein said material is glass.

In another embodiment, the present invention relates to the aforementioned microsphere, wherein the diameter of said microsphere is in the range from 5-75 micrometers.

In another embodiment, the present invention relates to the aforementioned microsphere, wherein the diameter of said microsphere is in the range from 5-500 micrometers.

In another embodiment, the present invention relates to the aforementioned microsphere, wherein the diameter of said microsphere is in the range from 10-100 micrometers.

In another embodiment, the present invention relates to the aforementioned microsphere, wherein the diameter of said microsphere is in the range from 20-50 micrometers.

In another embodiment, the present invention relates to the aforementioned microsphere, wherein said microsphere is solid, hollow, or comprises a plurality of hollow cells.

In another embodiment, the present invention relates to the aforementioned microsphere, wherein said microsphere is solid or hollow.

In another embodiment, the present invention relates to the aforementioned microsphere, wherein said microsphere is solid.

In another embodiment, the present invention relates to the aforementioned microsphere, wherein the density of said microsphere is in the range from 1.0-4.0 grams/cubic centimeter.

In another embodiment, the present invention relates to the aforementioned microsphere, wherein the density of said microsphere is in the range from 1.0-3.0 grams/cubic centimeter.

In another embodiment, the present invention relates to the aforementioned microsphere, wherein the density of said microsphere is in the range from 1.0-2.0 grams/cubic centimeter.

In another embodiment, the present invention relates to the aforementioned microsphere, wherein under mammalian physiological conditions said first radioisotope is not leached from said microsphere to an extent greater than 3%; wherein under mammalian physiological conditions said second radioisotope is not leached from said microsphere to an extent greater than 3%.

In another embodiment, the present invention relates to the aforementioned microsphere, wherein under mammalian physiological conditions said first radioisotope is not leached from said microsphere to an extent greater than 1%; wherein under mammalian physiological conditions said second radioisotope is not leached from said microsphere to an extent greater than 1%.

In another embodiment, the present invention relates to the aforementioned microsphere, wherein said first radioisotope is $^{90}$Y; and said second radioisotope is $^{198}$Au.

*Methods of the Invention*

The present invention also relates to a method of preparing a radioactive microsphere, comprising the steps of:

combining a non-radioactive precursor of a first radioisotope, a non-radioactive precursor of a second radioisotope, and a material selected from the group consisting of glass, polymer, and resin, to form a mixture; wherein said first radioisotope is $^{90}$Y or $^{32}$P; said second radioisotope is $^{198}$Au; and wherein the ratio of the radioactivity of the second radioisotope to the first radioisotope is in the range from 1:10 to $1:10^7$.

fabricating a microsphere from said mixture; and

bombarding said microsphere with neutrons.

In certain embodiments, the present invention relates to the aforementioned method and the attendant definitions, wherein said material is glass; said non-radioactive precursor of a first radioisotope is Y; and said non-radioactive precursor of a second radioisotope is Au.

Another aspect of the present invention relates to said microspheres for use in a method of treating a mammal suffering from a medical condition, comprising the step of:

administering to said mammal a therapeutically effective amount of radioactive microspheres each comprising a material selected from the group consisting of glass, polymer, and resin; a first radioisotope that emits a therapeutic $\beta$-particle; and a second radioisotope that emits a diagnostic $\gamma$-ray; wherein the atomic number of the first radioisotope is not the same as the atomic number of the second radioisotope.

In certain embodiments, the present invention relates to the aforementioned method and the attendant definitions, wherein the ratio of the radioactivity of the second radioisotope to the first radioisotope is in the range from about $1:10$ to about $1:10^7$ at the time of use.

In certain embodiments, the present invention relates to the aforementioned method and the attendant definitions, wherein the ratio of the radioactivity of the second radioisotope to the first radioisotope is in the range from about $1:10^2$ to about $1:10^6$ at the time of use.

In certain embodiments, the present invention relates to the aforementioned method and the attendant definitions, wherein the ratio of the radioactivity of the second radioisotope to the first radioisotope is in the range from about $1:10^4$ to about $1:10^5$ at the time of use.

In certain embodiments, the present invention relates to the aforementioned method and the attendant definitions, wherein said material is glass.

In certain embodiments, the present invention relates to the aforementioned method and the attendant definitions, wherein said first radioisotope is $^{90}Y$ or $^{32}P$.

In certain embodiments, the present invention relates to the aforementioned method and the attendant definitions, wherein said first radioisotope is $^{90}Y$.

In certain embodiments, the present invention relates to the aforementioned method and the attendant definitions, wherein said second radioisotope is $^{198}Au$.

In certain embodiments, the present invention relates to the aforementioned method and the attendant definitions, wherein said material is glass; said first radioisotope is $^{90}Y$ or $^{32}P$; and said second radioisotope is $^{198}Au$.

In certain embodiments, the present invention relates to the aforementioned method and the attendant definitions, wherein said material is glass; said first radioisotope is $^{90}Y$; and said second radioisotope is $^{198}Au$.

Said microspheres may be administered using a catheter or a syringe.

Said microspheres may be administered using a catheter.

## *Exemplification*

The invention now being generally described, it will be more readily understood by reference to the following examples, which are included for purposes of illustration of certain aspects and embodiments of the present invention.

## *Example 1*

### *Calculation of Optimal Amount of $^{197}Au$ Required for $^{89}Y$-containing Glass Microsphere Experimental Design*

In the present case, we wish to label radioactive $^{90}Y$-containing glass microspheres with a sufficient amount of $^{198}Au$ so that the microspheres are detectable by a gamma camera. For the purpose of the example, the microspheres are of the same composition as commercial Theraspheres (40% $Y_2O_3$ by weight, or 31 % Y), except for the presence of the gold compound. The process is to be carried out by neutron activating glass microspheres that contain the stable isotopes $Y^{89}$ and $Au^{197}$. In this example, we wish to compute the amount of initial $Au^{197}$ that is required, if the desired radioactivity at the time of removal of the sample from the neutron flux is to be 100 mCi of $Y^{90}$ and 1 $\mu$Ci of $Au^{198}$ per 50 mg of glass microspheres. The neutron capture cross-sections for $Y^{89}$ and $Au^{197}$ are 1.3 barns and 98.8 barns, respectively, and the decay constants for $Y^{90}$ and $Au^{198}$ are $3.01 \times 10^{-6}$ s$^{-1}$ and $2.98 \times 10^{-6}$s$^{-1}$, respectively. Supposing that the neutron flux is $1 \times 10^{14}$ cm$^{-2}$s$^{-1}$, the neutron capture constants are computed from Eq 4 to be $1.3 \times 10^{-10}$s$^{-1}$ for $Y^{89}$ and $9.88 \times 10^{-9}$s$^{-1}$ for $Au^{197}$. For both elements, the neutron capture constant is, to a good approximation, negligible compared to the decay constant. Under this circumstance, and as long as the neutron activation time (t) is less than about $5 \times 10^6$ s, Eq 6 is approximated by Eq 8, to within about 5%:

$$k_N = \phi\chi \qquad (\text{Eq 4})$$

$$\frac{dA^*}{dt} = -k_D A^* \qquad \text{(Eq 5)}$$

$$\frac{-dA^*}{dt} = \frac{k_D k_N A_0}{k_D - k_N} (e^{-k_N t} - e^{-k_D t}) \qquad \text{(Eq 6)}$$

$$\frac{-dA^*}{dt} \approx k_N A_0 (1 - e^{-k_D t}) \qquad \text{(Eq 8)}$$

Using Eq 8, we calculate that activating the 50 mg of glass microspheres (0.174 mmol of Y) to a radioactivity of 100 mCi will require about $1.05 \times 10^5$ s. Substituting values for the Au and Y isotopes into Eq 8 and forming ratios, and noting that the decay constants are nearly identical, we arrive at the final equation which expresses the ratio of radioactivity to the ratio of the initial amounts of stable isotopes:

$$\frac{d(Au^{198})/dt}{d(Y^{90})/dt} = \frac{\chi_{Au197} \, Au_0^{197}}{\chi_{Y89} \, Y_0^{89}} = \frac{76 \, Au_0^{197}}{Y_0^{89}} \qquad \text{(Eq 9)}$$

For the present example, substituting the desired radioactivity values in Eq 9 gives the starting mole ratio of gold to yttrium as:

$$\frac{Au_0^{197}}{Y_0^{89}} = 1.32 \times 10^{-7} \text{ (mole ratio)} = 2.92 \times 10^{-7} \text{ (mass ratio)}$$

In the glass composition, which is 31% Y by weight, the necessary amount of gold is finally computed to be 91 parts per billion, by weight.

### Example 2

In this example, we assume a glass composition containing 13% $Y_2O_3$ by weight (or 10% Y), and desire radioactivities of 100 mCi for $Y^{90}$ and 10 μCi for $Au^{198}$ per 50 mg of glass microspheres. The other quantities are as for Example 1. A similar computation shows that the glass should contain 291 ppb of gold, and requires a neutron activation time of $6.10 \times 10^5$ s. Similar calculations may be performed for other proportions of these elements or combinations of other elements in any proportions.

### Example 3

*Glass Bead Preparation*

The procedures for preparing glass microspheres has been reported previously. See U.S. Patent 5,302,369. In these preparations, glass of varying compositions of Si, Al, K, Mg, Al, Pb, and $P_2O_5$ has been prepared using reagent grade chemicals. Batches yielding 50 grams of glass were melted in platinum crucibles in an electric furnace at the approximate temperatures. A typical melting cycle required three hours for batch additions at 1000° C and three to four hours to refine the melt at the approximate melting temperature. The crucible containing the melt was quenched in 25° C water, after which the resultant glass frit was broken from the crucible and ground to -100 mesh. The -100 mesh glass powder was then slowly fed by a vibrating spatula into an oxygen/propane flame where surface tension pulled the molten particles into spheres. The flow rates of oxygen and propane were adjusted for each glass composition so as to yield the highest fraction of spherical particles. After spheroidizing, the microspheres were wet screened with deionized water, rinsed in acetone and dried.

## Claims

1. A microsphere, comprising a material selected from the group consisting of glass, polymer and resin; a first radio-isotope that emits a therapeutic β-particle; and a second radioisotope that emits a diagnostic γ-ray; wherein said first radioisotope is $^{90}$Y or $^{32}$P; said second radioisotope is $^{198}$Au; and the microsphere exhibits a ratio of the radioactivity of the second radioisotope to the first radioisotope in the range from 1:10 to 1:10$^7$.

2. The microsphere of claim 1, wherein the ratio of the radioactivity of the second radioisotope to the first radioisotope is in the range from 1: 10$^2$ to 1:10$^6$.

3. The microsphere of claim 1, wherein the ratio of the radioactivity of the second radioisotope to the first radioisotope is in the range from 1: 10$^4$ to 1: 10$^5$.

4. The microsphere of claim 1, wherein said material is selected from the group consisting of glass and polymer.

5. The microsphere of claim 1, wherein said material is glass.

6. The microsphere of claim 1, wherein the diameter of said microsphere is in the range from 5-75 micrometers.

7. The microsphere of claim 1, wherein the diameter of said microsphere is in the range from 5-500 micrometers.

8. The microsphere of claim 1, wherein the diameter of said microsphere is in the range from 10-100 micrometers.

9. The microsphere of claim 1, wherein the diameter of said microsphere is in the range from 20-50 micrometers.

10. The microsphere of claim 1, wherein said microsphere is solid, hollow, or comprises a plurality of hollow cells.

11. The microsphere of claim 1, wherein said microsphere is solid or hollow.

12. The microsphere of claim 1, wherein said microsphere is solid.

13. The microsphere of claim 1, wherein the density of said microsphere is in the range from 1.0-4.0 grams/cubic centimeter.

14. The microsphere of claim 1, wherein the density of said microsphere is in the range from 1.0-3.0 grams/cubic centimeter.

15. The microsphere of claim 1, wherein the density of said microsphere is in the range from 1.0-2.0 grams/cubic centimeter.

16. The microsphere of claim 1, wherein under mammalian physiological conditions said first radioisotope is not leached from said microsphere to an extent greater than 3%; wherein under mammalian physiological conditions said second radioisotope is not leached from said microsphere to an extent greater than 3%.

17. The microsphere of claim 1, wherein under mammalian physiological conditions said first radioisotope is not leached from said microsphere to an extent greater than 1%; wherein under mammalian physiological conditions said second radioisotope is not leached from said microsphere to an extent greater than 1%.

18. The microsphere of any one of claims 1-17, wherein said first radioisotope is $^{90}$Y.

19. A method of preparing a radioactive microsphere, comprising the steps of:

**EP 1 615 671 B1**

combining a non-radioactive precursor of a first radioisotope, a non-radioactive precursor of a second radioisotope, and a material selected from the group consisting of glass, polymer, and resin, to form a mixture; wherein said first radioisotope is $^{90}$Y or $^{32}$P; said second radioisotope is $^{198}$Au; and the microsphere exhibits a ratio of the radioactivity of the second radioisotope to the first radioisotope in the range from 1:10 to 1:10$^7$; fabricating a microsphere from said mixture; and bombarding said microsphere with neutrons.

20. The method of claim 19, wherein said material is glass; said nonradioactive precursor of a first radioisotope is Y; and said non-radioactive precursor of a second radioisotope is Au.

21. A microsphere as claimed in any of claims 1 to 18, for use in medicine.

22. A microsphere as claimed in any of claims 1 to 18, for use in treating cancer.

**Patentansprüche**

1. Mikrokügelchen umfassend ein Material ausgewählt aus der Gruppe bestehend aus Glas, Polymer und Harz; ein erstes Radioisotop, das ein therapeutisches β-Partikel emittiert; ein zweites Radioisotop, das diagnostische γ-Strahlen emittiert; worin das erste Radioisotop $^{90}$Y oder $^{32}$P ist; das zweite Radioisotop $^{198}$Au ist; und das Mikrokügelchen ein Verhältnis der Radioaktivität des zweiten Radioisotops zum ersten Radioisotop im Bereich von 1:10 bis 1:10$^7$ aufweist.

2. Das Mikrokügelchen gemäß Anspruch 1, worin das Verhältnis der Radioaktivität des zweiten Radioisotops zum ersten Radioisotop im Bereich von 1:10$^2$ bis 1:10$^6$ liegt.

3. Das Mikrokügelchen gemäß Anspruch 1, worin das Verhältnis der Radioaktivität des zweiten Radioisotops zum ersten Radioisotop im Bereich von 1:10$^4$ bis 1:10$^5$ liegt.

4. Das Mikrokügelchen gemäß Anspruch 1, worin das Material ausgewählt ist aus der Gruppe bestehend aus Glas und Polymer.

5. Das Mikrokügelchen gemäß Anspruch 1, worin das Material Glas ist.

6. Das Mikrokügelchen gemäß Anspruch 1, worin der Durchmesser des Mikrokügelchens im Bereich von 5-75 Mikrometern liegt.

7. Das Mikrokügelchen gemäß Anspruch 1, worin der Durchmesser des Mikrokügelchens im Bereich von 5-500 Mikrometern liegt.

8. Das Mikrokügelchen gemäß Anspruch 1, worin der Durchmesser des Mikrokügelchens im Bereich von 10-100 Mikrometern liegt.

9. Das Mikrokügelchen gemäß Anspruch 1, worin der Durchmesser des Mikrokügelchens im Bereich von 20-50 Mikrometern liegt.

10. Das Mikrokügelchen gemäß Anspruch 1, worin das Mikrokügelchen fest oder hohl ist oder ein Mehrzahl an hohlen Zellen aufweist.

11. Das Mikrokügelchen gemäß Anspruch 1, worin das Mikrokügelchen fest oder hohl ist.

12. Das Mikrokügelchen gemäß Anspruch 1, worin das Mikrokügelchen fest ist.

13. Das Mikrokügelchen gemäß Anspruch 1, worin die Dichte des Mikrokügelchens im Bereich von 1.0-4.0 g/cm$^3$ liegt.

14. Das Mikrokügelchen gemäß Anspruch 1, worin die Dichte des Mikrokogelchens im Bereich von 1.0-3.0 g/cm$^3$ liegt.

15. Das Mikrokügelchen gemäß Anspruch 1, worin die Dichte des Mikrokügelchens im Bereich von 1.0-2.0 g/cm$^3$ liegt.

16

**16.** Das Mikrokügelchen gemäß Anspruch 1, worin unter physiologischen Bedingungen von Säugern das erste Radioisotop nicht zu mehr als 3% aus dem Mikrokügelchen auswäscht; worin unter physiologischen Bedingungen von Säugern das zweite Radioisotop nicht zu mehr als 3% aus dem Mikrokügelchen auswäscht.

**17.** Das Mikrokügelchen gemäß Anspruch 1, worin unter physiologischen Bedingungen von Säugern das erste Radioisotop nicht zu mehr als 1 % aus dem Mikrokügelchen auswäscht; worin unter physiologischen Bedingungen von Säugern das zweite Radioisotop nicht zu mehr als 1% aus dem Mikrokügelchen auswäscht.

**18.** Das Mikrokügelchen gemäß einem der Ansprüche 1-17, worin das erste Radioisotop $^{90}$Y ist.

**19.** Verfahren zur Herstellung eines radioaktiven Mikrokügelchens, umfassend die Schritte von:

Kombinieren eines nichtradioaktiven Vorläufers eines ersten Radioisotops, eines nichtradioaktiven Vorläufers eines zweiten Radioisotops und ein Material ausgewählt aus der Gruppe bestehend aus Glas, Polymer und Harz, um eine Gemisch zu bilden; worin das erste Radioisotop $^{90}$Y oder $^{32}$P ist; das zweite Radioisotop $^{198}$Au ist; und das Mikrokügelchen ein Verhältnis der Radioaktivität des zweiten Radioisotops zum ersten Radioisotop im Bereich von 1:10 bis 1:10$^7$ aufweist;
Herstellen eines Mikrokügelchens aus dem Gemisch; und
Bombardieren des Mikrokügelchens mit Neutronen.

**20.** Das Verfahren von Anspruch 19, worin das Material Glas ist; der nichtradioaktive Vorläufer eines ersten Radioisotops Y ist; und der nichtradioaktive Vorläufer eines zweiten Radioisotops Au ist.

**21.** Ein Mikrokügelchen wie in einem der Ansprüche 1 bis 18 beansprucht zur Verwendung in der Medizin.

**22.** Ein Mikrokügelchen wie in einem der Ansprüche 1 bis 18 beansprucht zur Verwendung bei der Behandlung von Krebs.

**Revendications**

**1.** Microsphère, comprenant un matériau choisi dans le groupe constitué de verre, polymère et résine ; un premier radio-isotope qui émet une particule β thérapeutique ; et un deuxième radio-isotope qui émet un rayon γ diagnostique ; ledit premier radio-isotope étant $^{90}$Y ou $^{32}$P ; ledit deuxième radio-isotope étant $^{198}$Au ; et la microsphère présentant un rapport de la radioactivité du deuxième radio-isotope au premier radio-isotope dans la plage de 1:10 à 1:10$^7$.

**2.** Microsphère de la revendication 1, dans laquelle le rapport de la radioactivité du deuxième radio-isotope au premier radio-isotope est dans la plage de 1:20$^2$ à 1:10$^6$.

**3.** Microsphère de la revendication 1, dans laquelle le rapport de la radioactivité du deuxième radio-isotope au premier radio-isotope est dans la plage de 1:10$^4$ à 1:10$^5$.

**4.** Microsphère de la revendication 1, dans laquelle ledit matériau est choisi dans le groupe constitué du verre et d'un polymère.

**5.** Microsphère de la revendication 1, dans laquelle ledit matériau est du verre.

**6.** Microsphère de la revendication 1, dans laquelle le diamètre de ladite microsphère est dans la plage de 5 à 75 micromètres.

**7.** Microsphère de la revendication 1, dans laquelle le diamètre de ladite microsphère est dans la plage de 5 à 500 micromètres.

**8.** Microsphère de la revendication 1, dans laquelle le diamètre de ladite microsphère est dans la plage de 10 à 100 micromètres.

**9.** Microsphère de la revendication 1, dans laquelle le diamètre de ladite microsphère est dans la plage de 20 à 50 micromètres.

**10.** Microsphère de la revendication 1, ladite microsphère étant pleine, creuse, ou comprenant une pluralité de cellules creuses.

**11.** Microsphère de la revendication 1, ladite microsphère étant pleine ou creuse.

**12.** Microsphère de la revendication 1, ladite microsphère étant solide.

**13.** Microsphère de la revendication 1, dans laquelle la masse volumique de ladite microsphère est dans la plage de 1,0 à 4,0 grammes/centimètre cube.

**14.** Microsphère de la revendication 1, dans laquelle la masse volumique de ladite microsphère est dans la plage de 1,0 à 3,0 grammes/centimètre cube.

**15.** Microsphère de la revendication 1, dans laquelle la masse volumique de ladite microsphère est dans la plage de 1,0 à 2,0 grammes/centimètre cube.

**16.** Microsphère de la revendication 1, dans laquelle, dans des conditions physiologique pour un mammifère, ledit premier radio-isotope n'est pas lessivé depuis ladite microsphère à un degré supérieur à 3% ; dans laquelle, dans des conditions physiologique pour un mammifère, ledit deuxième radio-isotope n'est pas lessivé depuis ladite microsphère à un degré supérieur à 3 %.

**17.** Microsphère de la revendication 1, dans laquelle, dans des conditions physiologique pour un mammifère, ledit premier radio-isotope n'est pas lessivé depuis ladite microsphère à un degré supérieur à 1 % ; dans laquelle, dans des conditions physiologique pour un mammifère, ledit deuxième radio-isotope n'est pas lessivé depuis ladite microsphère à un degré supérieur à 1 %.

**18.** Microsphère de l'une quelconque des revendications 1 à 17, dans laquelle ledit premier radio-isotope est $^{90}$Y.

**19.** Procédé de préparation d'une microsphère radioactive, comprenant les étapes de :

combinaison d'un précurseur non radioactif d'un premier radio-isotope, un précurseur non radioactif d'un deuxième radio-isotope, et un matériau choisi dans le groupe constitué de verre, d'un polymère, et d'une résine, pour former un mélange ; dans lequel ledit premier radio-isotope est $^{90}$Y ou $^{32}$P ; ledit deuxième radio-isotope est $^{198}$Au ; et la microsphère présente un rapport de la radioactivité du deuxième radio-isotope au premier radio-isotope dans la plage de 1:10 à 1:10$^7$ ;
fabriquer une microsphère à partir dudit mélange ; et bombarder ladite microsphère avec des neutrons.

**20.** Procédé de la revendication 19, dans lequel ledit matériau est du verre ; ledit précurseur non radioactif d'un premier radio-isotope est Y ; et ledit précurseur non radioactif d'un deuxième radio-isotope est Au.

**21.** Microsphère selon l'une quelconque des revendications 1 à 18, pour utilisation en médecine.

**22.** A microsphère selon l'une quelconque des revendications 1 à 18, pour traiter le cancer.

**REFERENCES CITED IN THE DESCRIPTION**

**Patent documents cited in the description**

- US 6149889 A
- US 6328700 A
- US 5302369 A

**Non-patent literature cited in the description**

- **SCHEELE J ; ALTENDORF-HOFMANN A.** Resection of colorectal liver metastases. *Langenbeck's Arch. Surg.,* 1999, 313-327
- **HÄFELI UO ; CASILLAS S ; DIETZ DW ; PAUER GJ ; RYBICKI LA ; CONZONE SD ; DAY DE.** Hepatic tumor radioembolization in a rat model using radioactive rhenium (186Re/188Re) glass microspheres. *Int. J. Radiation Oncology Biol. Phys.,* 1999, vol. 44, 189-199
- **LINK KH ; KORNMAN M. ; FORMENTINI A ; LEDER G ; SUNELAITIS E ; SCHATZ M ; PRELMAR J ; BEGER HG.** Regional chemotherapy of non-resectable liver metastases from colorectal cancer - literature and institutional review. *Langenbeck's Arch. Surg.,* 1999, vol. 384, 344353
- **BASTIAN P ; BARTKOWSKI R ; KOHLER H ; KISSEL T.** Chemo-embolization of experimental liver metastases. Part 1: distribution of biodegradable microspheres of different sizes in an animal model for the locoregional therapy. *Eur. J. Pharm. Biopharm.,* 1998, vol. 46, 243-254
- **ACKERMAN NB ; LIEN WM ; KONDI ES ; SILVERMAN NA.** The blood supply of experimental liver metastases. The distribution of hepatic artery and portal vein blood to "small" and "large" tumors. *Surgery,* 1969, vol. 66, 1067-1072
- **NOLAN et al.** Intravascular Particulate Radioisotope Therapy. The American Surgeon, 1969, vol. 35, 181-188
- **GRADY et al.** Intra-Arterial Radioisotopes to Treat Cancer. American Surgeon, 1960, vol. 26, 678-684
- **ZIELINSKI ; KASPRZYK.** Synthesis and Quality Control Testing of P labelled Ion Exchange Resin Microspheres for Radiation Therapy of Hepatic Neoplasms. *Int. J. Appl. Radiat. Isot.,* 1983, vol. 34, 1343-1350
- **HO S ; LAU WY ; LEUNG TWT ; CHAN M ; NGAR YK ; JOHNSON PJ ; LI AKC.** Clinical evaluation of the partition model for estimating radiation doses from yttrium-90 microspheres in the treatment of hepatic cancer. *Eur. J Nucl. Med.,* 1997, vol. 24, 293-298
- **MUMPER RJ ; RYO UY ; JAY M.** Neutron activated holmium-166-Poly(L-lactic acid) microspheres: A potential agent for the internal radiation therapy of hepatic tumours. *J. Nucl. Med.,* 1991, vol. 32, 2139-2143
- **TURNER JH ; CLARINGBOLD PG ; KLEMP PFB ; CAMERON PJ ; MARTINDALE AA ; GLANCY RJ ; NORMAN PE ; HETHERINGTON EL ; NAJDOVSKI L ; LAMBRECHT RM.** Ho-microsphere liver radiotherapy: a preclinical SPECT dosimetry study in the pig. *Nucl. Med. Comm.,* 1994, vol. 15, 545-553
- **HO S ; LAU WY ; LEUNG TWT ; JOHNSON PJ.** Internal radiation therapy for patients with primary or metastatic hepatic cancer. *Cancer,* 1998, vol. 83, 1894-1907
- **SPENCER RP.** Applied principles of radiopharmaceutical use in therapy. *Nucl. Med. Biol.,* 1986, vol. 13, 461-463
- **SPENCER RP.** Short-lived radionuclides in therapy. *Nucl. Med. Biol.,* 1987, vol. 14, 537-538
- **CONZONE SD ; HÄFELI UO ; DAY DE ; EHRHARDT GJ.** Preparation and properties of radioactive rhenium glass microspheres intended for in vivo radioembolization therapy. *J. Biomed. Mater. Res.,* 1998, vol. 42, 617-625
- **BROWN RF ; LINDESMITH LC ; DAY DE.** 166-Holmium-containing glass for internal radiotherapy of tumors. *Int. J. Rad. Appl. Instrum. B,* 1991, vol. 18, 783-790
- **KAWASHITA M ; MIYAJI F ; KOKUBO T ; TAKAOKA GH ; YAMADA I ; SUZUKI Y ; INOUE M.** Surface structure and chemical durability of P+-implanted Y2O3-Al2O3-SiO2 glass for radiotherapy of cancer. *J. Non-Cryst. Solids,* 1999, vol. 255, 140-148
- **SCHUBIGER PA ; BEER H-F ; GEIGER L ; RÖSLER H ; ZIMMERMAN A ; TRILLER J ; METTLER D ; SCHILT, W.** Y-resin particles-animal experiments on pigs with regard to the introduction of superselective embolization therapy. *Nucl. Med. Biol.,* 1991, vol. 18, 305-311

- **ZIMMERMAN A ; SCHUBIGER PA ; METTLER D ; GEIGER L ; TRILLER J ; RÖSLER H.** Renal pathology after arterial yttrium-90 microsphere administration in pigs. A model for superselective radioembolization therapy. *Invest. Rad.,* 1995, vol. 30, 716-723
- **WANG S-J ; LIN W-Y ; CHEN M.-N ; CHI C-S ; CHEN J-T ; HO W-L ; HSIEH B-T ; SHEN L-H ; TSAI Z-T ; TING G.** Intratumoral injection of rhenium-188 microspheres into an animal model of hepatoma. *J Nucl. Med.,* 1998, vol. 39, 1752-1757
- **LAU WY ; LEUNG WT ; HO S ; LEUNG NWY ; CHAN M ; LIN J ; METREWELI C ; JOHNSON P ; LI AKC.** Treatment of inoperable hepatocellular carcinoma with intrahepatic arterial yttrium-90 microspheres: a phase I and II study. *Br. J. Cancer,* 1994, vol. 70, 994-999
- **LAU WY ; HO S ; LEUNG TWT ; CHAN M ; HO R ; JOHNSON PJ ; LI AKC.** Selective internal radiation therapy for nonresectable hepatocellular carcinoma with intraarterial infusion of yttrium microspheres. *Int. J. Radiation Oncology Biol. Phys.,* 1998, vol. 40, 583592
- **WUNDERLICH G ; PINKERT J ; ANDREEFF M ; STINTZ M ; KNAPP FF ; KROPP J ; FRANKE WG.** Preparation and biodistribution of rhenium-188 labeled albumin microspheres B 20: a promising new agent for radiotherapy. *Appl. Radiat. Isotopes,* 2000, vol. 52, 63-68
- **RHODES BA ; ZÖLLE I ; BUCHANAN JW ; WAGNER HN.** Radioactive albumin microspheres for studies of the pulmonary circulation. *Radiology,* 1969, vol. 92, 1453-1460
- **WATANABE N ; ORIUCHI N ; ENDO K ; INOUE T ; TANADA S ; MURATA H ; SASAKI Y.** Yttrium-90 labeled human macroaggregated albumin for internal radiotherapy: combined use with DTPA. *Nucl. Med. Biol.,* 1999, vol. 26, 847-851
- **MUMPER RJ ; JAY M.** Poly(L-lactic acid) microspheres containing neutron-activatable holmium-165: A study of the physical characteristics of microspheres before and after irradiation in a nuclear reactor. *Pharm. Res.,* 1992, vol. 9, 149-154
- **HÄFELI UO ; SWEENEY SM ; BERESFORD BA ; HUMM JL ; MACKLIS RM.** Effective targeting of magnetic radioactive Y-microspheres to tumor cells by an externally applied magnetic field. Preliminary in vitro and in vivo results. *Nucl. Med. Biol.,* 1995, vol. 22, 147-155
- **O'DONNELL PB ; MCGINITY JW.** Preparation of microspheres by solvent evaporation technique. *Adv. Drug Del. Rev.,* 1997, vol. 28, 25-42
- **GRAY BN ; BURTON MA ; KELLEHER D ; KLEMP P ; MATZ L.** Tolerance of the liver to the effects of yttriuin-90 radiation. *Int. J. Radiation Oncology Biol. Phys.,* 1990, vol. 18, 619-623
- **TIAN J-H ; XU B-X ; ZHANG J-M ; DONG B-W ; LIANG P ; WANG X-D.** Ultrasoundguided internal radiotherapy using yttrium-90-glass microspheres for liver malignancies. *J. Nucl. Med.,* 1996, vol. 37, 958-963
- **HO S ; LAU WY ; LEUNG TWT ; CHAN M ; CHAN KW ; LEE WY ; JOHNSON PJ ; LI AKC.** Tumour-to-normal ratio of Y microspheres in hepatic cancer assessed with Tc macroaggregated albumin. *Brit. J. Rad.,* 1997, vol. 70, 823-828
- **YORKE ED ; JACKSON A ; FOX RA ; WESSELS BW ; GRAY N.** Can current models explain the lack of liver complications in Y-90 microsphere therapy?. *Clin. Cancer Res.,* 1999, vol. 5, 3024s-3030s
- **INGOLD J ; REED G ; KAPLAN H ; BAGSHAW M.** Radiation hepatitis. *Am. J. Roentgenol. Radium Ther. Nucl. Med.,* 1965, vol. 93, 200-208
- **VAN ES RJJ ; FRANSSEN O ; DULLENS HFJ ; BERNSEN MR ; BOSMAN F ; HENNINK WE ; SLOOTWEG PJ.** The VX2 carcinoma in the rabbit auricle as an experimental model for intra-arterial embolization of head neck squamous cell carcinoma with hydrogel dextran microspheres. *Lab. Anim.,* 1999, vol. 33, 175-184
- **TOMURA N ; KATO K ; HIRANO H ; HIRANO Y A ; WATARAI J.** Chemoembolization of maxillary tumors via the superficial temporal artery using a coaxial catheter system. *Radiation Med.,* 1998, vol. 16, 157
- **ARIEL IM ; PADULA G.** Irradiation of the spleen by the intra-arterial administration of yttrium microspheres in patients with malignant lymphoma. *Cancer,* 1972, vol. 31, 90-96
- Webster's New World Dictionary. 1984
- Handbook of Chemistry and Physics. 1986